(19) Europäisches Patentamt
European Patent Office
Office européen des brevets

(11) Publication number: 0 254 329 B1

(12) # EUROPEAN PATENT SPECIFICATION

(45) Date of publication of the patent specification: 26.09.90

(51) Int. Cl.[5]: C07D 311/24, A61K 31/35

(21) Application number: 87112296.6

(22) Date of filing: 18.01.84

(60) Publication number of the earlier application in accordance with Art. 76 EPC: 0139809

(54) **Substituted dihydrobenzopyranones.**

(30) Priority: 08.08.83 US 520973
12.12.83 US 560355

(43) Date of publication of application: 27.01.88 Bulletin 88/4

(45) Publication of the grant of the patent: 26.09.90 Bulletin 90/39

(84) Designated Contracting States: BE CH DE FR GB IT LI NL SE

(56) References cited: EP-A- 0 079 637

(73) Proprietor: G.D. Searle & Co., P.O. Box 1045, Skokie Illinois 60076(US)

(72) Inventor: Miyano, Masateru, 2139 Valley Road, Northbrook, IL 60062(US)
Inventor: Shone, Robert L., 1441 Joan Drive, Palatine, IL 60067(US)
Inventor: Sohn, Daniel D., c/o Lundgren Jakosbergs Platsen 6A, S-72461 Vuasteris(SE)

(74) Representative: Wolff, Hans Joachim, Dr.jur. Dipl.-Chem. et al, Beil, Wolff & Beil Rechtsanwälte
Postfach 80 01 40 Adelonstrasse 58, D-6230 Frankfurt am Main 80(DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 254 329 B1

**Description**

BACKGROUND OF THE INVENTION

a) Field of the Invention

This invention in its broadest aspect, relates to metabolic inhibitors. In particular the invention relates to novel compounds of Formula I which are inhibitors of leukotriene $D_4$ ($LTD_4$) and therefore useful to prevent or alleviate the symptoms or conditions associated with $LTD_4$ such as allergic reactions and inflammatory conditions.

$LTD_4$ is a product of the 5-lipoxygenase pathway and is the major active constituent of slow reacting substance of anaphylaxis (SRS-A) in humans and guinea pigs, Lewis etal., Nature USA, 293: 103-108, (1981). It is a potent bronchoconstrictor that is released during allergic reactions. Because antihistamines are ineffective in the management of asthma it is believed that SRS-A mediates bronchoconstriction resulting from an allergic attack. SRS-A is also a potent inducer of vascular permeability, and it also may be involved in other inflammatory conditions such as rheumatoid arthritis, Geller, J., et al., J. Clin. Endocrinol. Metab. 43: 686-688,(1976).

b) Information Disclosure

Appleton et al., J. Med. Chem. 20, 371-379 (1977) discloses a series of chromone-2-carboxylic acids which are antagonists of SRS-A. Specifically sodium 7-[3-(4-acetyl- 3-hydroxy-2-propylphenoxy)-2-hydroxypropoxy]-4-oxo-8-propyl-4H-1-benzopyran-2-carboxylate (FPL 55712), appears to be the first reported specific antagonist of SRS-A and $LTD_4$. EP-A-0 079 637 discloses anti-SRS-A carboxylic acid derivatives some of which being structurally similar to those of the claimed compounds.

EP-A-0 129 906 which is an older application according to Article 54(3) EPC discloses phenoxyalkoxy-3,4-dihydro-2H-1-benzopyran derivatives which differ in some respects from the claimed substituted dihydrobenzopyrans.

SUMMARY OF THE INVENTION

The invention relates to compounds of the formula:

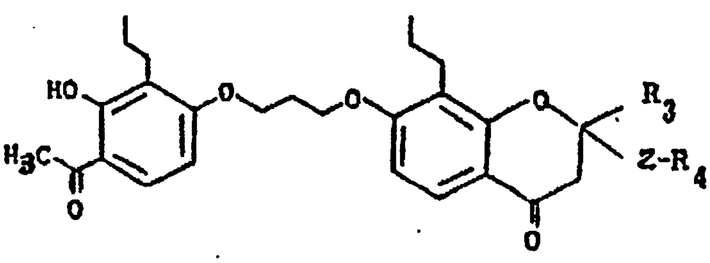

I

wherein $R_3$ and $R_4$ are $COOR_5$; wherein Z is $-CH_2-CH_2-$, and wherein $R_5$ is hydrogen or lower alkyl having 1-6 carbon atoms,
or the pharmacologically acceptable addition salts thereof.

Examples of alkyl of 1 to 6 carbon atoms inclusive are methyl, ethyl, propyl, butyl, pentyl, hexyl, and the isomeric forms thereof.

Salts of the acid forms of these compounds can be prepared by neutralization with the appropriate amount of an inorganic or organic base such as sodium hydroxide, potassium hydroxide, calcium hydroxide, magnesium hydroxide, ammonia, trialkylamine, dialkylamine, monoalkylamine, dibasic amino acids, sodium acetate, potassium benzoate, triethanolamine and like bases.

$LTD_4$ causes bronchoconstriction when administered to humans and guinea pigs. The bronchoconstriction has 2 components: a) a direct stimulation of respiratory smooth muscle by $LTD_4$ and b) an indirect component through release of thromboxane A2 which in turn causes contraction of respiratory smooth muscle. Compounds of the invention antagonize the direct component. The compounds are tested in vivo as follows.

Adult male fasted Hartly guinea pigs weighing 300-350g are pretreated with pyrilamine and indomethacin to block the bronchoconstricture effects of endogenous histamine and the synthesis of thromboxane A2 respectively. Compounds of the invention are administered IV or IG at appropriate times prior to the IV administration of 2000 units of $LTD_4$. Intratracheal pressure is monitored prior to and subsequent to $LTD_4$ in animals anesthetized with pentobarbital and attached to a rodent respirator. Compounds

which antagonize the direct component of $LTD_4$ action on respiratory smooth muscle inhibit intratracheal insufflation pressure increases (P or = 0.05) caused by $LTD_4$. FPL 55712 is used as a control.

The compounds can be administered in a number of dosage forms. A preferred method of delivery would be oral or in such a manner so as to localize the action of the inhibitor. For example, for asthma, the compounds could be inhaled using an aerosol or other appropriate spray. In an inflammatory condition such as rheumatoid arthritis the compounds could be injected directly into the affected joint. The compounds could also be administered in oral unit dosage forms such as tablets, capsules, pills, powders or granules. They also may be administered rectally or vaginally in such forms as suppositories. They may be introduced in the forms of eyedrops, intraperitoneally, subcutaneously, or intramuscularly using forms known to the pharmaceutical art. For the treatment of inflammatory allergic skin conditions, the compounds of the present invention may also be administered topically in the form of ointments, creams, gels or the like. Regardless of the route of administration selected, the compounds are formulated into pharmaceutically acceptable dosage forms by conventional methods known to the pharmaceutical art.

An effective but non-toxic quantity of the compound is employed in treatment. The dosage regimen for inhibition of $LTD_4$ by the compounds of this invention is selected in accordance with a variety of factors including the type, age, weight, sex, and medical condition of the mammal, the particular disease and its severity, the route of administration and the particular compound employed. An ordinarily skilled physician or veterinarian will readily determine and prescribe the effective amount of the compound to prevent or arrest the progress of the condition. In so proceeding, the physician or veterinarian could employ relatively low dosages at first, subsequently increasing the dose until a maximum response is obtained.

The compounds of this invention are prepared by the general methods illustrated in Charts A to C. The various compounds and intermediates can be readily modified by methods known to those skilled in the art. For example, esters can be hydrolyzed to corresponding carboxylic acids (and their respective addition salts), converted to corresponding amides by appropriate reactions with amines, and reduced to alcohols by such reagents as Lithium borohydride. Such products and intermediates can, of course, be similarly interconverted.

As illustrated in Chart A, 2-hydroxyacetophenones of Formula XI react readily with ketones of Formula XII to afford fused ring compounds of Formula XIII Ketones, Formula XII, include ketoesters in which Z is ethylene $R_4$ is alkoxycarbonyl. An example of such a ketoester is ethyl levulinate. Preferred condensation and cyclization conditions include heating Formulas XI and XI at reflux in toluene, in the presence of a base such as pyrrolidine, with provisions for the removal of water with an apparatus such as a Dean-Stark trap. Intermediates of Formula XIII thus formed are used in reactions of Chart B.

As illustrated in Chart B, compounds of Formula XIII may be alkylated under basic conditions to form compounds of Formula XXI. Preferred reagents include dihaloalkanes, such as 1,3-dibromopropane and the like. Preferred conditions include reaction in dry dimethylformamide in the presence of anhydrous potassium carbonate. Intermediates XXI are typically purified by column chromatography on silica gel. Reaction of these intermediates with 2-hydroxyacetophenones of Formula XXII afford title compounds of this invention, Formula XXIII. Preferred conditions include reaction in dry dimethylformamide in the presence of anhydrous potassium carbonate. Alternatively, the reaction may be run under phase transfer conditions.

Chart C illustrates preparation of compounds XXIII using a variation of the method of Chart B. 2-Hydroxyacetophenones of Formula XXII react with dihaloalkanes as described above (See Chart B) to form intermediates of Formula XXXI. By the same general procedure employed in converting Formula XIV to Formula XXI, compounds XXXI and XIV react to form the title compounds of this invention, Formula XXIII.

DESCRIPTION OF THE PREFERRED EMBODIMENTS

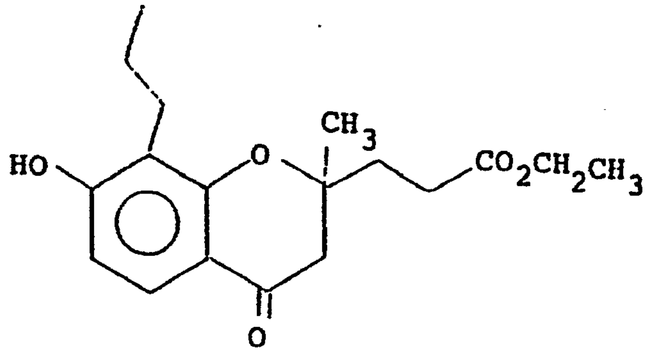

Preparation 1 ethyl 3-(3,4-dihydro-7-hydroxy-2-methyl-4-oxo-8-propyl-2H-1-benzopyran-2-yl)propanoate

A solution of 58.0g (0.299 mole) of 2,4-dihydroxy-3-propylacetophenone and 29.5ml (0.36 mole) of pyrrolidine in 250ml of toluene was heated to reflux for three hours under a Dean-Stark trap. After cooling the mixture, 68.2ml (0.48 mole) of ethyl levulinate was added and the mixture was refluxed for two hours. An additional 10ml (0.12 mole) of pyrrolidine was added and the mixture was refluxed overnight under a Dean-Stark trap.

The reaction mixture was diluted with ethyl acetate and washed successively with water, 2 M hydrochloric acid, water and brine. It was then dried over $MgSO_4$, filtered, and evaporated to dryness. The residue was chromatographed on silica gel using 30% ethyl acetate/hexane as eluent to give 35.5g of the title ester, mp. 92-94°. Structure assignment was confirmed by nmr and infrared spectra.
IR($CHCl_3$): 1665, 1730 $cm^{-1}$

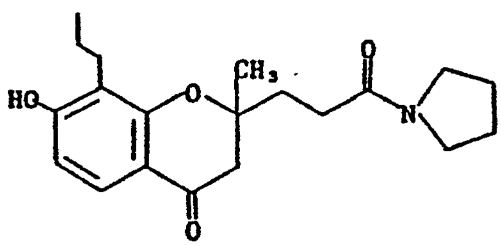

Preparation 2 1-[3-(3,4-dihydro-7-hydroxy-2-methyl-4-oxo-8-propyl-2H-1-benzopyran-2-yl)-1-oxopropyl] pyrrolidine

Other chromatographic fractions of Example 1 afforded 30 g of the title amide, m.p. 182-184°. Structure assignment was confirmed by nmr and infrared spectra and by elemental analysis.
IR(KBr): 1615, 1675 $cm^{-1}$
Analysis. Calcd. for $C_{20}H_{27}NO_4$: C, 69.54; H, 7.88; N, 4.05.
Found: C, 69.22; H, 7.93; N, 3.97.

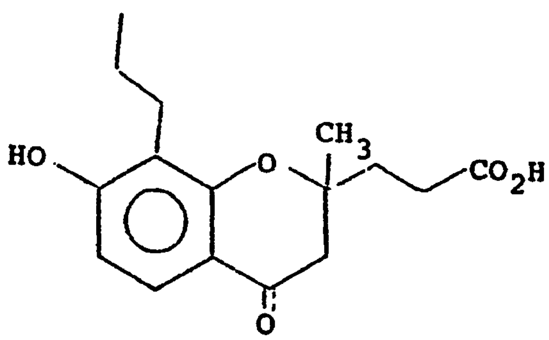

Preparation 3 3-(3,4-dihydro-7-hydroxy-2-methyl-4-oxo-8-propyl-2H-1-benzopyran-2-yl)propanoic acid

To a mixture of 20.0 g (103 mmole) of 2,4-dihydroxy-3-propylacetophenone and 15.5 g (134 mmole) of methyl levulinate in 100 ml of dry toluene was added by syringe 21.5 ml (ca. 260 mmole) of pyrrolidine. After stirring for one hour the solution was heated on a steam bath for four hours and allowed to cool. The toluene solution was washed with water and 2 N NaOH. The basic solution was acidified and extracted with diethyl ether, which in turn was extracted with saturated sodium bicarbonate. The basic aqueous extract was neutralized with dilute hydrochloric acid and again extracted with diethyl ether. After drying, the solution was concentrated at reduced pressure to an oil which crystallized to the title compound, m.p. 152-153°.

Analysis calcd. for $C_{16}H_{20}O_5$: C, 65.74; H, 6.90.
Found: C. 65.24; H, 6.86.

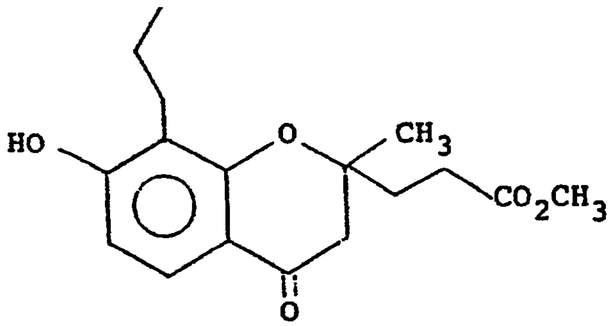

Preparation 4 methyl 3-(3,4-dihydro-7-hydroxy-2-methyl-4-oxo-8-propyl-2H-1-benzopyran-2-yl)propanoate

A mixture of the title compound of Preparation 3 (4.3 g, 14.7 mmole), 6 ml of trimethylorthoformate, and 1.4 ml of sulfuric acid was stirred at room temperature for two hours. The reaction mixture was poured onto stirred ice/water and extracted with ethyl acetate. The organic phase was washed with water, dried over magnesium sulfate, and concentrated to an oil. The oil was purified by chromatography on silica gel, giving 3.7 g of the title compound as a crystalline solid, m.p. 101.5-102.5°. Structure assignment was consistent with nmr and infrared spectra.

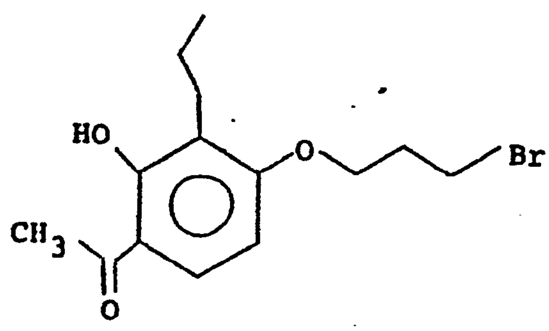

Preparation 5 4-(3-bromopropoxy)-2-hydroxy-3-propylacetophenone

To a mixture of 50.0 g (257 mmole) of 2,4-dihydroxy-3-propylacetophenone, 87.4 g (257 mmole) of tetrabutylammonium hydrogen sulfate, and 52.2 ml (ca. 514 mmole) of dibromopropane in 250 ml of dichloromethane was added 225 ml (450 mmole) of 2 N NaOH. The mixture was then heated at reflux for about 30 min. allowed to cool. After concentrating the reaction mixture under vacuum, the residue was triturated with diethyl ether, and the ether solution was filtered and concentrated to dryness. After purification by column chromatography on silica gel, the title compound was isolated and used in subsequent reactions without further characterization.

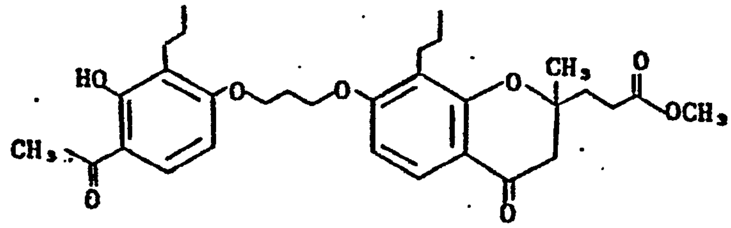

Preparation 6 methyl 3-[7-[3-(4-acetyl-3-hydroxy-2-propylphenoxy)propoxy]-3,4-dihydro-2-methyl-4-oxo-8-propyl-2H-1-benzopyran-2-yl]propanoate

A mixture of 1.9g (6.2 mmole) of the ester product of Preparation 4, 2.2g (6.8 mmole) of the title product of Preparation 5, and 1.8g (13 mmole) of anhydrous potassium carbonate in 30ml of dry dimethylformamide was stirred for 16 hours at room temperature. The inorganic salts were removed by filtration and the dimethylformamide was evaporated invacuo. The residue was dissolved in ethyl acetate and additional inorganic salts were filtered. The filtrate was evaporated and the residue was chromatographed on silica gel using 7% ethyl acetate/toluene as eluent to afford 2.5g of the title compound, mp. 73-74.5°. Structure assignment was confirmed by nmr and infrared spectra and by elemental analysis.
nmr ($CDCl_3$): δ(ppm) 0.89 (t, J=7.5Hz, 6H, propyl $CH_3$'s); 1.24 (t, J=7Hz, 3H, ester $CH_3$); 1.35 (s, 3H, 2-methyl protons); 2.55 (s, 3H, acetyl $CH_3$); 4.22 (t, J=6Hz, 4H, $OCH_2$'s); 6.42, 6.53, 7.54, 7.71 (sets of d's, aromatic)
IR($CHCl_3$): 1625, 1675, 1730 $cm^{-1}$
Analysis. Calcd. for $C_{31}H_{40}O_8$: C, 68.87; H, 7.46.
Found: C, 69.12; H. 7.44.

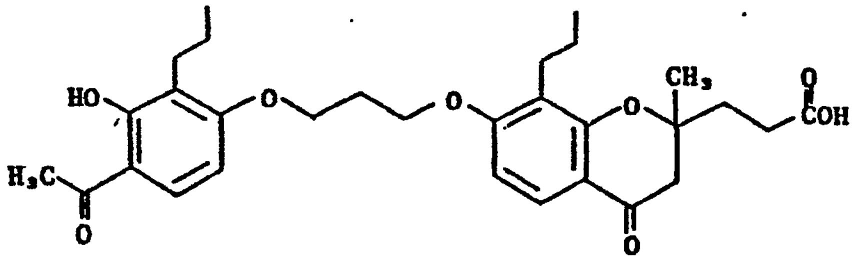

Preparation 7 3-[7-[3-(4-acetyl-3-hydroxy-2-propylphenoxy)propoxy]-3,4-dihydro-2-methyl-4-oxo-8-propyl-2H-1-benzopyran-2-yl]propanoic acid

A solution of 0.5g (19.5 mmole) of lithium hydroxide in 10ml of water was added to a solution of 2.2g (3.9 mmole) of the title compound of Preparation 6 in 25ml of tetrahydrofuran and 20ml of methanol. After the reaction mixture was stirred for 3 hours at room temperature, the solvent was evaporated and water was added to the residue. After extracting with a small amount of diethyl ether, the aqueous layer was acidified with dilute hydrochloric acid and allowed to stand in the cold overnight. The solid was filtered, washed with water and recrystallized from ethyl acetate/hexane to yield 1.83g of the title compound, mp. 80-83°. Structure assignment was confirmed by nmr and infrared spectra and by elemental analysis.
nmr ($CDCl_3$): loss of ester $CH_3$ (compare Preparation 6)
IR(KBr): 1625, 1675, 1710, 1730 $cm^{-1}$
Analysis. Calcd. for $C_{31}H_{38}O_8 \cdot H_2O$: C, 66.16; H, 7.40.
Found: C, 66.15; H, 7.44.

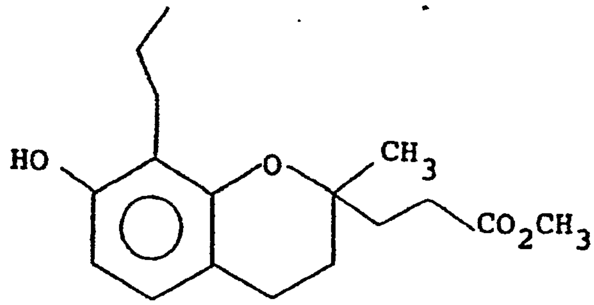

Preparation 8 methyl 3-(3,4-dihydro-7-hydroxy-2-methyl-8-propyl-2H-1-benzopyran-2-yl)propanoate

A solution of 4.1g (13.4 mmole) of ester product of Preparation 4 in 100ml of acetic acid and 50ml of methanol was hydrogenated for 21 hours at 4 psi and room temperature using palladium on carbon as catalyst. The catalyst was filtered and the filtrate was evaporated to dryness. Chromatography of the residue on silica gel using 20% ethyl acetate/hexane as eluent gave 1.8g of the title compound as an oil. Structure assignment was confirmed by nmr and infrared spectra.
nmr $CKCl_3$): δ(ppm) 0.94 (t, J=7Hz, 3H, propyl $CH_3$); 1.23 (s, 3H, 2-methyl protons); 3,67 (s, 3H, ester $CH_3$); 6.28 (d, J=8Hz, 1H, aromatic); 6.71 (d, J=8Hz, 1H, aromatic)
IR($CHCl_3$) 1730 $cm^{-1}$

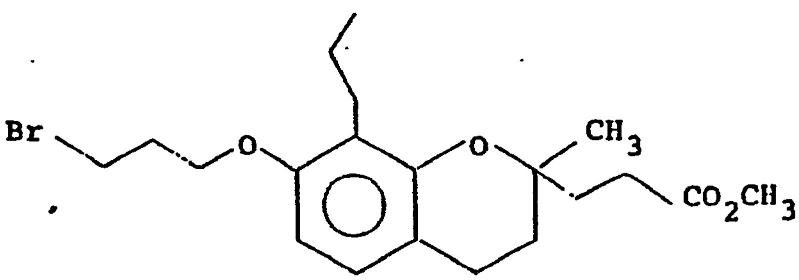

Preparation 9 methyl 3-[7-(3-bromopropoxy)3,4-dihydro-2-methyl-8-propyl-2H-1-benzopyran-2-yl] propanoate

To a solution of 1.7g (5.51 mmole) of the title compound from Preparation 8, 1.1ml (11 mmoles) of 1,3-dibromopropane and 1.9g (11 mmole) of tetrabutylammonium hydrogen sulfate in 12ml of methylene chloride was added 5.5ml of 2 M sodium hydroxide solution. The reaction mixture was heated to reflux for 15 minutes and cooled. The organic layer was separated, washed with brine, dried over sodium sulfate, filtered, and concentrated to dryness. Chromatography of the residue on silica gel using 10% ethyl acetate/hexane as eluent gave 1.8g of the title compound as an oil. Structure assignment was confirmed by nmr and infrared spectra.

nmr ($CDCl_3$): addition of $CH_2Br$ signal at δ(ppm) 4.02 (t, J=6Hz) (compare Preparation 8)
IR($CHCl_3$): 1735 $cm^{-1}$

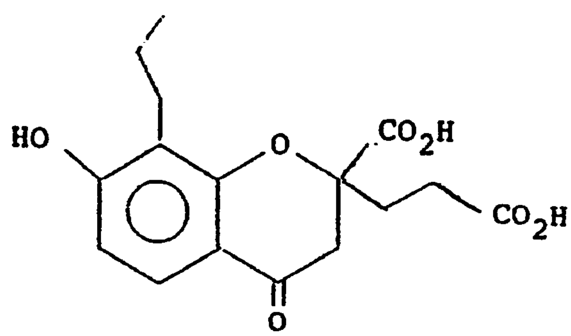

Preparation 10 3-(2-carboxy-3,4-dihydro-7-hydroxy-4-oxo-8-propyl-2H-1-benzopyran-2-yl)propanoio acid

The title compound was prepared by the method of Preparation 1 using α-ketoglutaric acid as starting material. The crude product was used without further purification. α-ketoglutaric acid can be replaced with homolegues, for instance, 4-ketopinslic acid to produce homologous of the title compound.

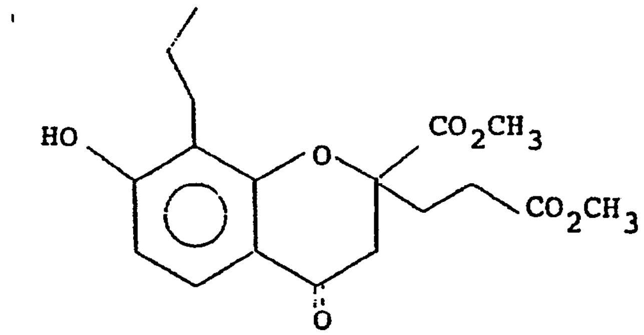

Preparation 11 methyl 3-(3,4-dihydro-7-hydroxy-2-methoxy carbonyl-4-oxo-8-propyl-2H-1-benzopyran-2-yl) propanoate

To a solution of 13g of the crude product of Preparation 10 in 250ml of methanol and 25ml of trimethylorthoformate was added 5ml of sulfuric acid. The reaction mixture was stirred for 12 hours at room temperature, poured onto an ice/water mixture, and extracted with ethyl acetate. The organic layer was washed successively with water, 5% sodium bicarbonate solution, water and brine, dried over $MgSO_4$, filtered, and evaporated to dryness. Chromatography of the residue on silica gel using 13% ethyl acetate/hexane as eluent gave 6.1g of the title compound. Structure assignment was confirmed by nmr and infrared spectra.

nmr ($CDCl_3$) δ(ppm) 0.99 (t, J=6Hz, 3H, propyl $CH_3$); 3.64, 3.71 (pair s, 6H, ester $CH_3$'s); 6.45, 7.56 (pair d's, aromatic)
IR($CHCl_3$): 1685, 1740 $cm^{-1}$

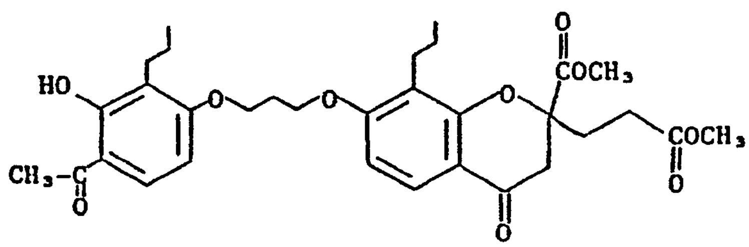

Example 1 methyl 3-[7-(3-(4-acetyl-3-hydroxy-2-propyl phenoxy)propoxy]-3,4-dihydro-2-methoxy carbonyl-4-oxo-8-propyl-2H-1-benzopyran-2-yl]propanoate

The title compound was prepared by the method of Preparation 2 using the product of Preparation 9 as starting material. Structure assignment was confirmed by nmr and infrared spectra and by elemental analysis substituting appropriate compounds homologs where I is 1 or 2 may be made.
nmr ($CDCl_3$): δ(ppm) 0.88, 0.93 (pair t, 6H, propyl $CH_3$'s); 2.56, (s, 3H, acetyl $CH_3$); 3.61, 3.68 (pair s, 6H, ester $CH_3$'s) 4.21 (t, J=7Hz, 4H, $OCH_2$'s); 6.39, 6.55, 7.53, 7.66 (sets of d's, aromatic)
IR($CHCl_3$): 1630, 1685, 1740 $cm^{-1}$
Analysis. Calcd. for $C_{32}H_{40}O_{10}$: C, 65.74; H, 6.90.
Found: C, 65.89; H. 6.87.

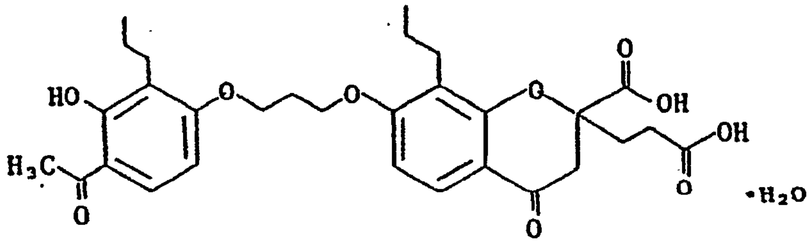

Example 2 3-[7-[3-(4-acetyl-3-hydroxy-2-propylphenoxy) propoxy]-2-carboxy-3,4-dihydro-4-oxo-8-propyl-2H-1-benzopyran-2-yl]propanoic acid

The title compound, mp. 76-79°, was prepared by the method of Preparation 7 using the product of Example 1 as starting material, except that ten equivalents of lithium hydroxide were used. The acidified aqueous layer was extracted into ethyl acetate, which was then washed with water and brine, dried over $MgSO_4$, filtered, and evaporated to dryness to give the title compound. Structure assignment was confirmed by nmr and infrared spectra and by elemental analysis.
nmr ($CDCl_3$): loss of ester $CH_3$'s (compare Example 16)
IR(KBr): 1620, 1680, 1710-1740 $cm^{-1}$
Analysis. Calcd. for $C_{30}H_{36}O_{10} \cdot H_2O$: C, 62.71; H, 6.67.
Found: C, 62.85; H. 6.64.

## CHART A

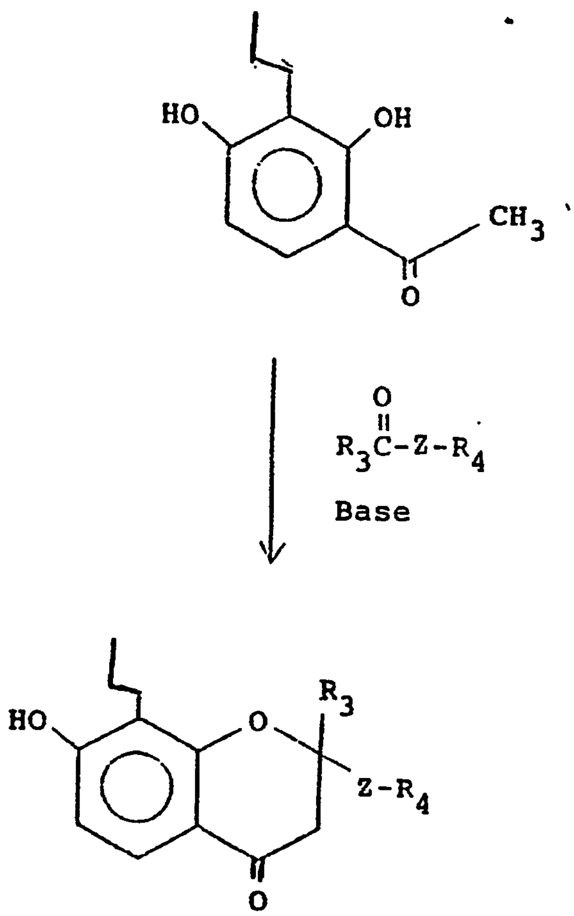


XI

XII

XIII

## CHART B

XIII

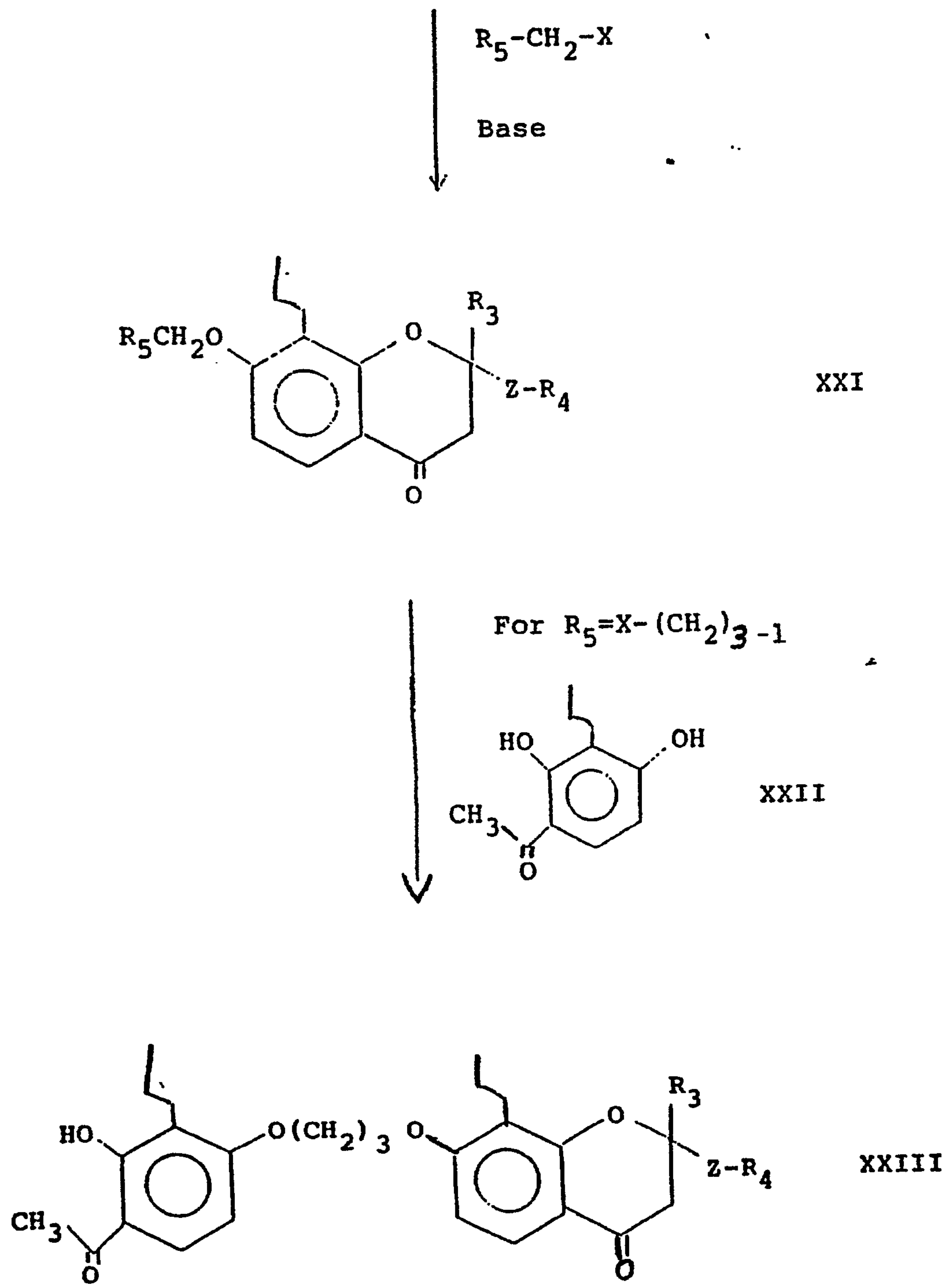


XXI

XXII

XXIII

## CHART C

XXII

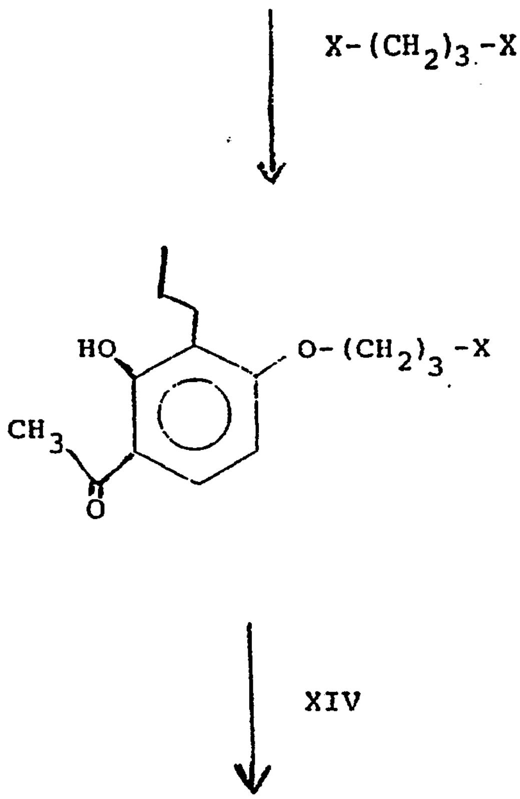


XXXI

XXIII

## Claims

1. A compound of the formula

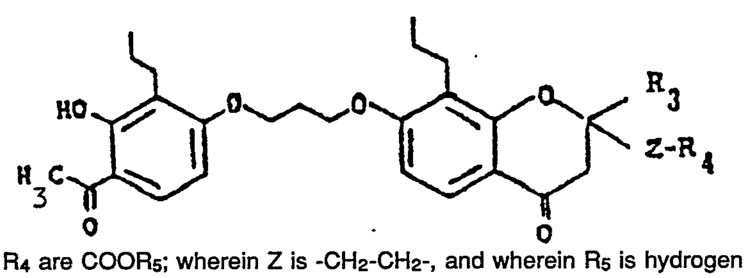

I

wherein $R_3$ and $R_4$ are $COOR_5$; wherein Z is $-CH_2-CH_2-$, and wherein $R_5$ is hydrogen or lower alkyl having 1-6 carbon atoms,
or the pharmacologically acceptable addition salts thereof.

2. A compound according to Claim 1 of the formula

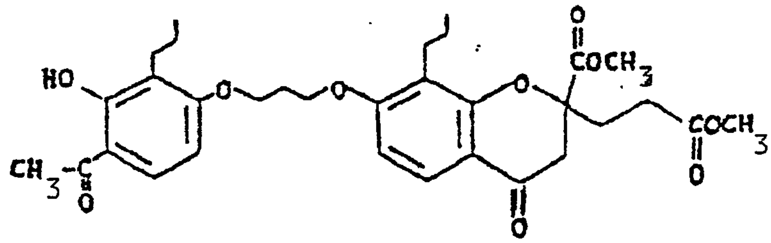

3. A compound according to Claim 1 of the formula

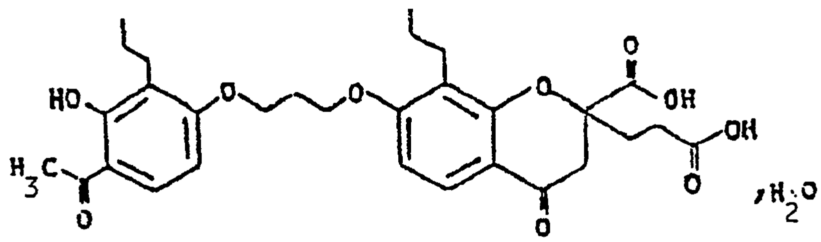

## Patentansprüche

1. Eine Verbindung der allgemeinen Formel

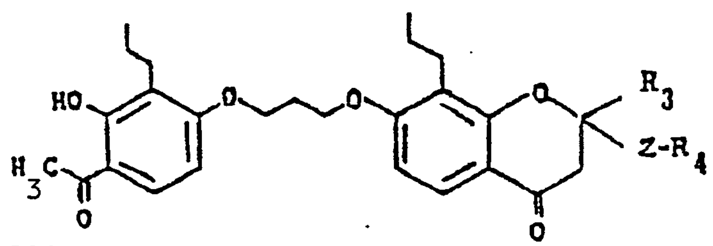

I

worin $R_3$ und $R_4$ $COOR_5$ bedeuten, Z $-CH_2-CH_2-$ ist und $R_5$ Wasserstoff oder eine Alkylgruppe mit 1–6 Kohlenstoffatomen darstellt, oder deren pharmazeutisch zulässigen Additionssalze.

2. Eine Verbindung gemäß Anspruch 1 der Formel

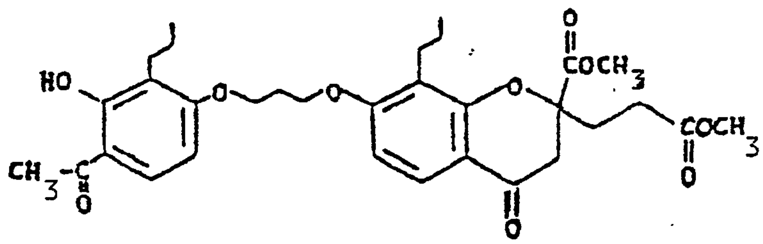

3. Eine Verbindung gemäß Anspruch 1 der Formel

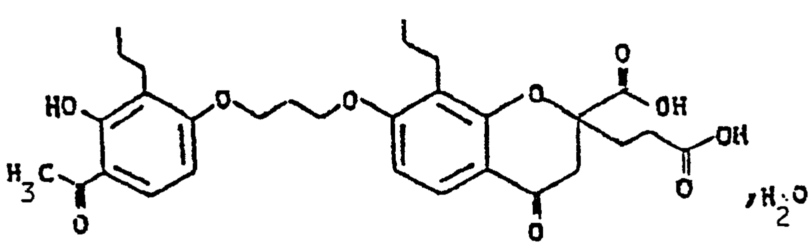

## Revendications

1. Un composé de formule

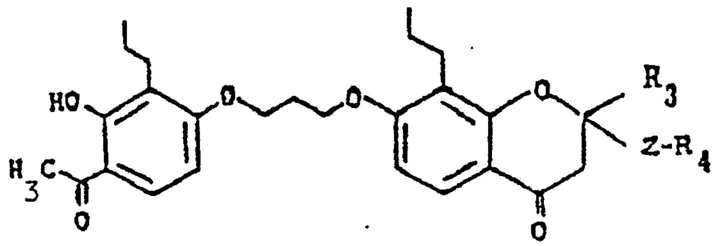

I

dans laquelle $R_3$ et $R_4$ désignent le groupe $COOR_5$; Z est le groupe $-CH_2-CH_2-$ et $R_5$ est un atome d'hydrogène ou un groupe alkyle inférieur ayant 1–6 atomes de carbone, ou les sels d'addition pharmacologiquement acceptables dudit composé.

2. Un composé selon la revendication 1 de formule

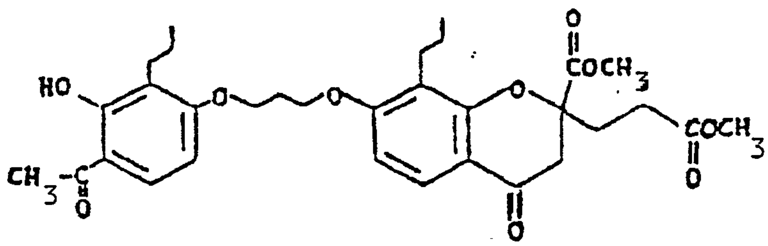

3. Un composé selon la revendication 1 de formule